# EUROPEAN PATENT SPECIFICATION

(11) **EP 1 318 768 B1**
(45) Date of publication and mention of the grant of the patent: **31.12.2008**
(21) Application number: 01966464.8
(22) Date of filing: 30.08.2001
(51) Int. Cl.: A61F 2/06

(54) **Intravascular stent consisting of stairstep expansion strut pairs and double stairstep diagonal connecting struts**
Blutgefässstütze bestehend aus gestuften, aufweitbaren Stegepaaren und doppelt gestuften, schrägen Verbindungsstegen
Stent intravasculaire constitué de paires de tiges d'extension en forme d'escalier et de tiges de liaisons diagonales en forme d'escalier à deux marches

(30) Priority: 23.09.2000 US 235164 P; 28.08.2001 US 942077
(43) Date of publication of application: 18.06.2003
(73) Proprietor: Boston Scientific Scimed, Inc., Maple Grove, MN 55311 (US)
(72) Inventor: JANG, G. David, Redlands, CA 92374 (US)
(74) Representative: Hauck Patent- und Rechtsanwälte
(86) International application number: PCT/US2001/027158
(87) International publication number: WO 2002/024109

(56) References cited:
- WO-A-00/30563
- US-A- 6 039 756
- US-A- 6 113 627

## Description

### Field of Invention:

This invention relates generally to intravascular stents, and more particularly to intra-coronary stents that provide intraluminal-scaffolding support of a vascular wall after percutaneous angioplasty in which a balloon catheter is used to expand a stenotic vascular lesion.

### Description of the Related Art:

By the year 2000, the percutaneous balloon angioplasty and stent implant procedures have become the dominant non-surgical revascularization method of the atherosclerotic stenosis, or obstruction, of the vascular lumen, and particularly in the coronary vascular system of the heart. With balloon angioplasty alone, without use of stent, the restenosis rate after angioplasty has been as high as 25-45% in the first time coronary cases. With use of stents after balloon angioplasty, the restenosis has been reduced significantly. Even so, the restenosis rate after stent implant is reported to be 15-25% range in coronary arteries, depending on the condition of the vessel stented or what specific stent was used. An ideal coronary stent is still elusive in the current state of the art commercial products.

Some of the best selling current, second generation, stents can be divided into two categories. One category is a stent with flexibility and the other with good vessel coverage. The flexible current stents generally have poor vessel coverage, tissue prolapse, rough surface modulation and increased restenosis rate. On the other hand, a good vessel coverage stent in the current state of art has better vessel coverage but not flexible enough for easy delivery and efficient procedure. This means that an ideal stent that has good flexibility and good vessel coverage remains as the gold standard that has not yet been reached.

To further reduce the restenosis rate after stent implant, numerous means has been tried, including laser, atherectomy, high frequency ultrasound, radiation device, local drug delivery, etc. Although the brachytherapy (radiation treatment) has proved to be reasonably effective in further reducing restenosis after stent implant, using brachytherpy is very cumbersome, inconvenient and costiy. Mainly because it is radioactive device and radiation therapy specialist from another department has to be involved with the interventional cardiologist in the cardiac catheterization laboratory. The laser and atherectomy devices proved to be marginally useful in this purpose with added costs. Even if these measures would reduce the restenosis rate in theory or in real terms, an ideal stent that has good vessel coverage and flexibility would produce even better outcomes.

The local drug therapy appears to be a very promising method for the future, as better pharmaceutical, chemical or biogenetic agents are developed and became available. Some research data, both from animal tests and human clinical studies, indicate that there are evidences of suppressing restenosis after stent implant when certain growth blocking pharmaceutical agents available today are used to coat the stent. In another instances, it has been speculated that certain surface modifying materials coated on the surface of the stent may be beneficial by it alone or in combination with growth suppressing agent, in reducing restenosis rate. In either instance, the drug or substance should be locally attached or coated on the stent and in sufficient amounts. However, attaching or coating a sufficient amount of a substance or drug on the coronary stent is not so easy a proposition, because coating enough volume of the drug on the small surface area of the stent is an elusive dream. If and when stent coating becomes practical, an ideally designed stent would still have better outcomes than a poorly designed stent, when used with substance coating.

US-A-6113627 discloses a circumferentially connected stent in a non-expanded state with a longitudinal axis, including a plurality of expansion struts forming a first expansion column. The first expansion column includes a first expansion strut, a second expansion strut and a first joining strut. The first joining strut couples a distal end of the first expansion strut to a distal end of the second expansion strut, and the first expansion strut has a stepped distal portion and the second expansion strut has a stepped proximal portion. A plurality of expansion struts defines a second expansion column, and the second expansion column includes a first expansion strut, a second expansion strut and a first joining strut which couples a distal end of the first expansion strut to a distal end of the second expansion strut, and the first expansion strut has a stepped proximal portion and the second expansion strut have a stepped distal portion. A first serial connecting strut column is formed of a plurality of serial connecting struts and includes a first serial connecting strut. The first serial connecting strut column couples the first expansion column to the second expansion column.

There is a need for an improved stent that is very flexible and substantially fully covers a vessel surface inside a vascular lumen.

### SUMMARY OF THE INVENTION

Accordingly, an object of the present invention is to provide a stent that is very flexible and substantially fully covers vessel surfaces inside a vascular lumen.

Another object of the present invention is to provide an improved stent that is easily delivered with a delivery balloon to a selected vascular lumen site.

Still another object of the present invention is to provide a stent that provides good flexibility, a smooth surface modulation without tulips and sufficient radiopacity during the delivery phase of the stent.

A further object of the present invention is to provide a stent with good flexibility, a smooth surface modulation without tulips and sufficient radiopacity in the delivery and post delivery phase to the selected vascular site.

These and other objects of the present invention are achieved in a stent, in a non-expanded state, that includes a first expansion column with individual expansion struts which form a plurality of expansion strut pairs. Each two adjacent expansion strut pairs in the first expansion column share a common strut. A second expansion column has individual expansion struts that form a plurality of expansion strut pairs. Each two adjacent expansion strut pairs in the second expansion column share a common strut. A first connecting strut column includes a plurality of individual connecting struts. Each individual connecting strut is an extension arm of an individual expansion strut from the first expansion column, and an extension arm of an individual expansion strut of the second expansion column. Each connecting strut forms a double stair-step pattern.

### BRIEF DESCRIPTION OF DRAWINGS

- Figure 1: is a side-elevation view of one embodiment of an un-expanded stent of the present invention.
- Figure 2: is anisometric view of the Figure 1 stent drawn in scale for a 15 mm length.
- Figure 3: is a close up view of the first and second expansion columns.
- Figure 4: is a close up view of the Figure 1 stent.
- Figure 5: illustrates one embodiment of the connecting struts of the first connecting strut column.
- Figure 6: illustrates one embodiment of the connecting struts that form the second connecting strut column, connector structs joining the expansion struts of Figure 4.
- Figure 7: is a close up view of closed cells created by adjacent expansion columns and their associated connecting strut colums.
- Figure 8: is illustrates the alignment of two adjacent expansion columns.

### DETAILED DESCRIPTION OF THE PREFERRED EMBODIMENTS

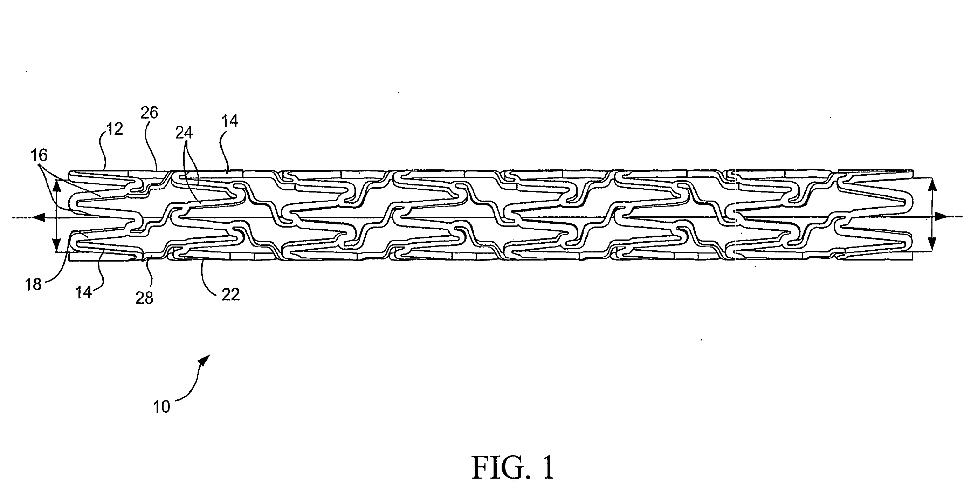

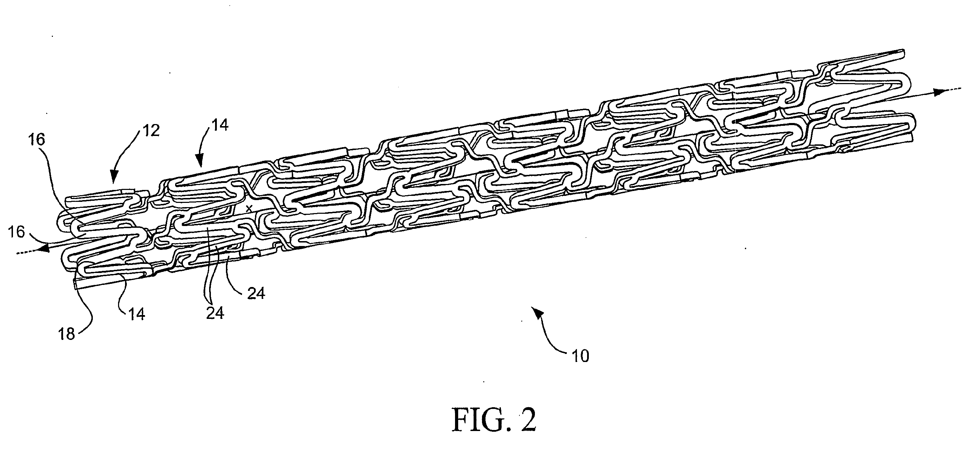

Referring now to Figures 1 and 2, one embodiment of a stent 10 of the present invention is illustrated in a non-expanded state. Stent 10 includes a first expansion column 12 with individual expansion struts 14 that form a plurality of expansion strut pairs 16. Adjacent expansion strut pairs 16 in first expansion column 12 share a common strut, indicated as 18. A second expansion column 20 also has individual expansion struts 22 that form a plurality of expansion strut pairs 22. Adjacent expansion strut pairs 22 in second expansion column 20 share a common strut, indicated as 24. Stent 10 is configured to provide easy delivery that is achieved with a sufficient level of flexibility in combination with a delivery balloon, a smooth surface modulation without tulips and a reasonable radiopacity during the delivery phase of stent 10.

Stent 10 provides enhanced flexibility and conformability while maintaining a full vessel coverage with optimal metal fraction. Additionally, stent 10 has, (i) substantially evenly expanding stent struts, (ii) excellent radial strength and radiopacity and (iii) smooth surface modulations in both the delivery and deployed phases of the stent life cycle. Stent 10 has a continuous, unbroken cylindrical form without any break or de-linking around the circumference and along its length.

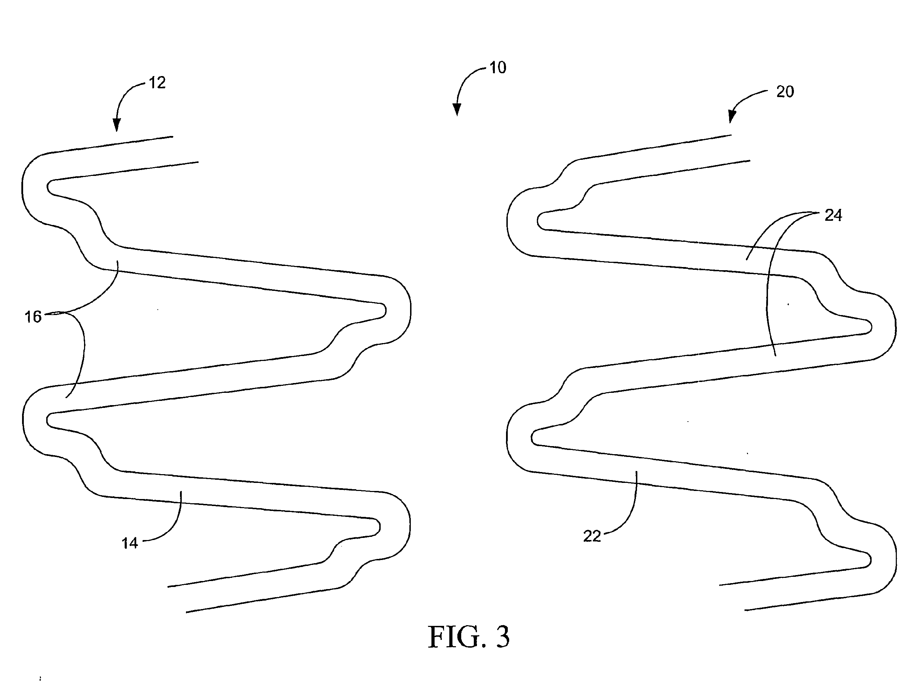

As illustrated in Figure 3, expansion strut pairs 16 and 24 form loops that couple adjacent individual expansion struts 14 and 22. In one embodiment, the loops of expansion strut pairs 16 and 24 are aligned in a peak to valley geometry. In another embodiment, the loops of expansion strut pairs 16 and 24 are aligned in a peak to peak geometry.

Expansion strut pairs 16 and 24 are jointed by a joining strut segment on distal and proximal ends and form the loops to form a "zigzig" pattern that continues for a selected number of cycles without a break around a circumference of stent 10. The number of cycles can be any number but in one embodiment the number is six or less.

Expansion struts 14 and 22 have first and second segments. At least a portion of the first segment of expansion struts 14 is positioned in close proximity in front of the loop of an expansion strut pair 16. At least a portion of the second section of expansion struts 22 is positioned in close proximity in front of the loop of an expansion strut pair 24. In one embodiment, close proximity is a distance of at least 0,0254 mm (0.001 inch). In another embodiment, close proximity is a distance less than 0,0254 mm (0.04 inch). At least one expansion strut 14 and 22 of expansion strut pairs 16 and 24 can have a stair-step segment at its proximal end and, the other expansion strut of the expansion strut pairs 16 and 24 has a stair-step segment at its distal end. In various embodiments, expansion struts 14 and 22 can have a, (i) short stepped-down segment at the proximal end, (ii) short stepped-down segment at the distal end, (iii) short stepped-up segment at the proximal end and short stepped-up segment at a the distal end. In all of these embodiments, expansion struts 14 and 22 have a short sloped transitional segment of that can have the same length between the long and short parts in expansion struts 14 and 22.

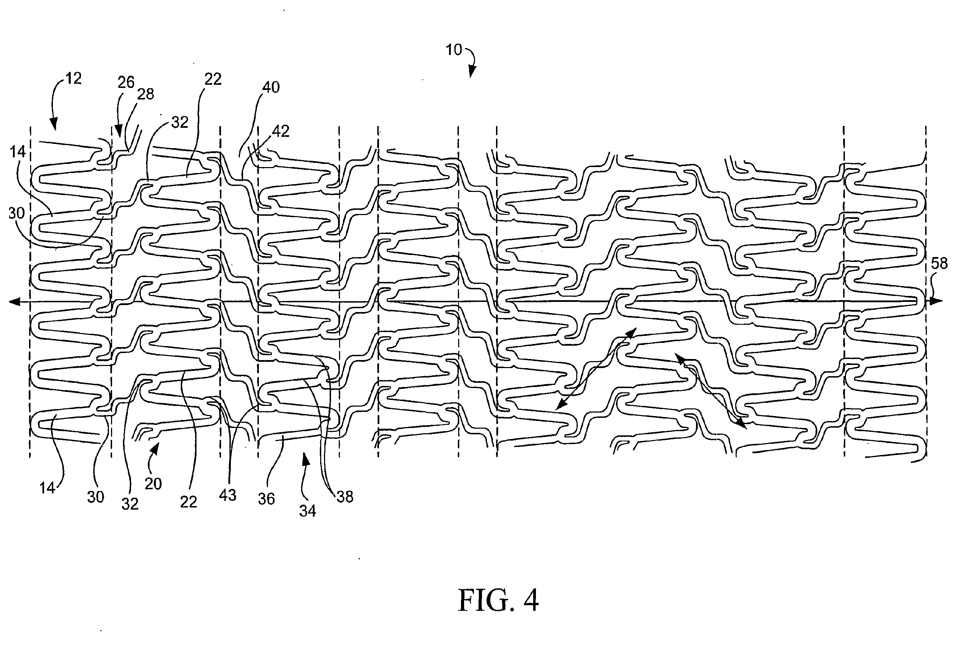

As illustrated in Figure 4, stent 10 includes a first connecting strut column 26 with a plurality of individual connecting struts 28. Each connecting strut 28 is an extension arm 30 of an expansion strut 14 from first expansion column 12, and an extension arm 32 of an expansion strut 22 of second expansion column 20.

In the Figure 4 embodiment, expansion columns 12, 20, 34, and the others in stent 10, have six zigzag cycles. Each six cycle expansion column includes twelve horizontally arranged expansion struts Connecting columns 26, 40 and the others do not have a zigzag cycle, but have doulbe stair-step connecting struts that are arranged in a paralleling alignment. For every one pair of expansion struts, there is only one associated connecting strut and the ratio of expansion struts to connecting struts is two-one.

Stent 10 also includes additional expansion and connecting strut columns. Stent 10 includes a third expansion column 34 with expansion struts 36 that form expansion strut pairs 38. A second connecting strut column 40 is made of a plurality of individual connecting struts 42. Each connecting strut 42 is an extension arm 43 of an expansion strut 22 from second expansion column 20 and an extension arm 44 of an expansion strut 36 of third expansion column 34.

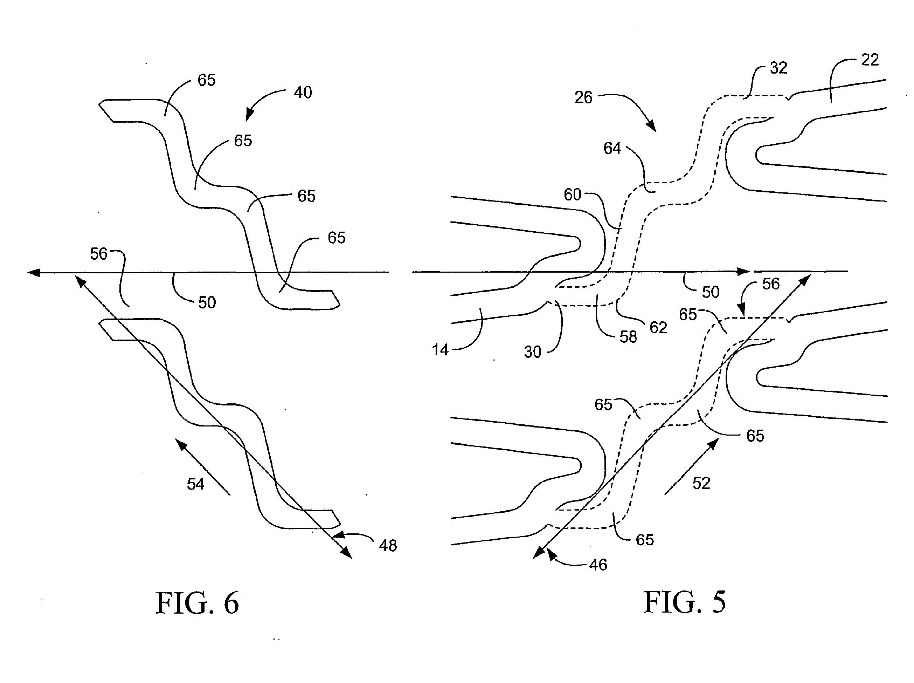

In various embodiments, one or both extension arms 30 and 32 extend from contra-lateral sides or ipsi-lateral sides of two opposing expansion strut pairs 16 and 24 (Figure 5); and one or both extension arms 32 and 46 extend from contra-lateral sides or ipsi-lateral sides of two opposing expansion strut pairs 24 and 38 respectively. Extension from contra-lateral sides provides a diagonal link pathway and multiple angled pivot points of a connecting strut 28 and 42 provides enhanced flexibility, conformability and excellent crimping characteristics to stent 10. Connecting struts 28 in first connecting strut column 26 have a longitudinal axis 46 (Figure 5) and connecting struts 42 in second connecting strut column 40 have a longitudinal axis 48 (Figure 6) that is non-parallel to longitudinal axis 46. In various embodiments, longitudinal axis 46 and 48 can be, (i) non-perpendicular to a longitudinal axis 50 of stent 10, (ii) substantially perpendicular to longitudinal axis 50, (iii) substantially diagonal in angle with respect to longitudinal axis 50 and (iv) substantially parallel to longitudinal axis 50.

Longitudinal axis 46 extends in one direction 52 while longitudinal axis 48 extends in an opposite direction 54. Longitudinal axis 46 and 48 each have a diagonal angle 56 with respect to a longitudinal axis of stent 10. Diagonal angle 56 of longitudinal axis 46 extends in direction 52 in any number of different patterns, while diagonal angle 56 of longitudinal axis 48 extends in direction 54 in any number of different patterns. Substantially all of the connecting struts 28 in first connecting strut column 26 have a parallel longitudinal axis 46. The same is true with every connecting strut 42 in second connecting strut column 40, as well as other connecting struts in other connecting strut columns. Preferably, every connecting strut 42 in first connecting strut column 26 has the same diagonal angle 56 with respect to longitudinal axis 50. The same is true of all other connecting struts in the other connecting strut columns of stent 10. Each longitudinal axis 46 and 48 has a slant angle vertical configuration, e.g. with diagonal angle 56, relative to longitudinal axis 50 of stent 10. This slant angle vertical configuration enhances the flexibility of stent 10 and is crimping characteristics on a balloon.

In all the embodiments of the present invention, connecting struts 28 between the first 12 and the second 20 expansion columns form a double stair-step pattern. Any other connecting struts in additional connecting strut columns form a (i) double stair-step pattern (Figures 5 and 6), (ii) multiple stair-step pattern, (iii) a stair-step pattern. Step-pattern may include at east one substantially horizontal segment 58 and at least one substantially slant-angled segment 60 and (iv) at least one substantially horizontal segment 58, at least one substantially slant-angled segment 60 and a curved section 62 that joins substantially horizontal segment 58 with substantially slant angled segment 60 (v) first segment 58 and a second segment 64, with at least a portion of first segment 58 is positioned in close proximity to a loop of an expansion strut pair 16 in first expansion column 12. Curved section 62 can have one radius of curvature, multiple radii of curvature, variable degrees radius or radii or curvature, a wide or a narrow radius of curvature.

In the Figure 5 and 6 embodiments, expansion struts 14 and 22 have double stair-step patterns with multiple angled pivot points 65. Pivot points 65 also enhance the flexibility of stent 10. Additionally, linking first and second expansion columns 12 and 20 in a diagonally manner relative to longitudinal axis 50 provides further flexibility to stent 10.

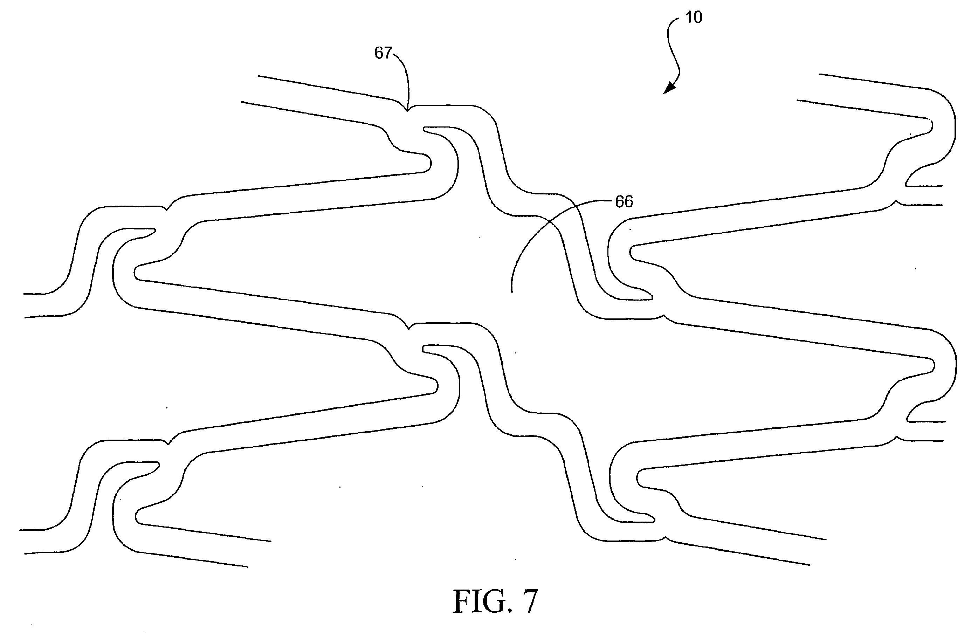

Adjacent expansion columns and their associated connecting strut columns define a plurality of cells 66 that are illustrated in Figure 7. Cells 66 have asymmetrical or symmetrical geometries. Cells 66 can have evenly spaced geometric shapes throughout stent 10. In one embodiment, cells 66 have substantially six sides when stent 10 is in a nominally expanded state. In another embodiment, cells 66 have substantially hexagonal geometric configurations when stent 10 is in a nominally expanded state. Optionally included are strain relief notches 67 that relieve the strain caused by metal deformation when stent 10 is expanded in the deployment phase.

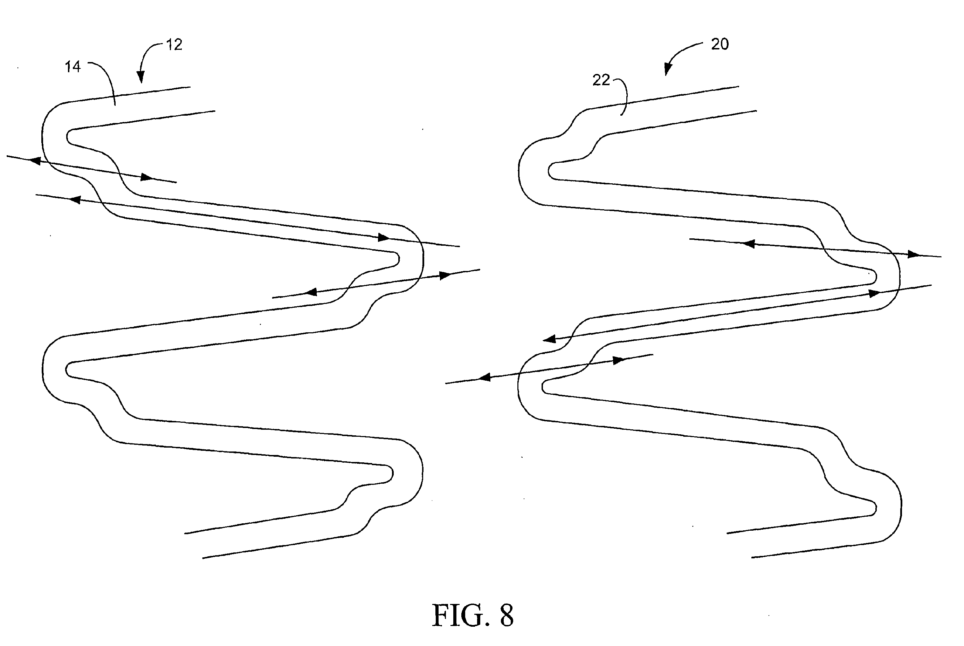

Figure 8 illustrates one embodiment of the spacing and alignment characteristics of expansion columns 12 and 20 without illustrating first connecting strut column 26. In this embodiment, the width of first connecting strut column 26 is narrower than the width of expansion columns 12 and 20. However, the width of first connecting strut column 26 can be narrower, the same as or wider than the width of expansion columns 12 and 20. Additionally, the width of any connecting strut column in stent 10 can be variable and different from one or more of the other connecting strut columns of stent 10. Further, the width of any expansion column in stent 10 can be different and variable from one or more other expansion columns in stent 10.

First, second and third expansion columns 12, 20 and 34 can each form a corrugated expansion ring.

## Claims

1. A stent (10) in a non-expanded state, comprising:
• a first expansion column (12) including individual expansion struts (14) forming a plurality of expansion strut pairs (16), wherein two adjacent expansion strut pairs (16) share a common strut (18);
• a second expansion column (20) including individual expansion struts (22) forming a plurality of expansion strut pairs (24), wherein two adjacent expansion strut pairs (24) share a common strut,
• a first connecting strut column (26) including a plurality of individual connecting struts (28), wherein each of an individual connecting strut (28) is an extension arm (30) of an individual expansion strut (14) from the first expansion column (12) and an extension arm (32) of an individual expansion strut (22) of the second expansion column (20),
**characterized in that** each connecting strut (28) forms a double stair-step pattern.

2. The stent of claim 1, wherein at least one end of a connecting strut (28) is an extension arm of an expansion strut from the first (12) or second (20) expansion column.

3. The stent of claim 1, wherein each end of a connecting strut (28) is an extension arm of an expansion strut, expending from contra-lateral sides of two opposing expansion strut pairs (16,24) of the first (12) and second (20) expansion columns.

4. The stent of claim 1, wherein at least one end of a connecting strut (28) is an extension arm of an expansion strut, expending from contra-lateral side or ipsi-lateral side of one of two opposing expansion strut pairs (16,24) of the first (12) and second (20) expansion columns.

5. The stent of claim 1, wherein each connecting strut (28) has a longitudinal axis that is non-perpendicular to a longitudinal axis of the stent (10); or
• is substantially perpendicular to a longitudinal axis of the stent (10); or
• is substantially diagonal in angle with respect to a longitudinal axis of the stent (10); or
• is substantially parallel to a longitudinal axis of the stent (10).

6. The stent of claim 1, wherein each connecting strut (28) has a longitudinal axis, and substantially every longitudinal axis of a connecting strut (28) in the first connecting column (26) is parallel to the others in the first connecting column (26).

7. The stent of claim 1, wherein each longitudinal axis of a connecting strut (28) in a connecting strut column (26) is parallel to the longitudinal axes of other connecting struts in the same connecting strut column (26) at a diagonal angle with respect to a longitudinal axis of the stent (10).

8. The step of claim 1, wherein each connecting strut (28) forms at least one stair-step pattern that includes at least one substantially horizontal segment and at least one substantially slant-angled segment.

9. The stent of claim 1, wherein each connecting strut (28) forms a stair-step pattern with at least one substantially horizontal segment, at least one substantially slant-angled segment and a curved section that joins the substantially horizontal segment and the substantially slant angled segment, the curved section having at least one or two radii of curvature, preferably
a radius of curvature at a junction between a substantially horizontal segment and
a substantially slant-angled segment has a wide angle radius of curvature or a narrow angle radius of curvature.

10. The stent of claim 1, further comprising:
• a third expansion column (34) including individual expansion struts (36) forming a plurality of expansion strut pairs (38), wherein two adjacent expansion strut pairs share a common strut;
• a second connecting strut column (40) including a plurality of individual connecting struts (42), wherein each of an individual connecting strut (42) is an extension arm (43) of an individual expansion strut (22) from the second expansion column (20) and an extension arm (43) of an individual expansion strut (36) of the third expansion column (34) and optionally,
• a plurality of expansion columns coupled by a plurality of connecting strut columns, each of a connecting strut in a connecting strut column having a longitudinal axis, wherein the longitudinal axis of the connecting struts in a connecting strut column extend in an opposite direction relative to the longitudinal axis of connecting struts in an adjacent connecting strut column.

11. The stent of claim 10, wherein each connecting strut (28) in the first connecting strut column (26) has a first longitudinal axis and each connecting strut (42) in the second connecting strut column (40) has a second longitudinal axis that is non-parallel to the first longitudinal axis.

12. The stent of claim 10, wherein the longitudinal axis of each of a connecting strut in a connecting strut column has a diagonal angle with respect to the longitudinal axis of the stent, each longitudinal axis of the connecting struts in a connecting strut column having a diagonal angle in an opposite direction relative to a diagonal angle of each longitudinal axis of the connecting strut in an adjacent connecting strut columns.

13. The stent of claim 10, wherein a diagonal angle of the longitudinal axis of each connecting strut in one connecting strut column having opposite direction in an alternating pattern in relation to a diagonal angle of the longitudinal axis of each connecting strut in an adjacent connecting strut column of a plurality of consecutive connecting strut columns.

14. The stent of claim 1, wherein the first expansion column (12), the second expansion column (20) and the first connecting strut column (26) define a plurality of cells (66).

15. The stent of claim 14, wherein the cells have asymmetrical geometry; or the cells (66) have evenly spaced geometric shapes throughout the stent, preferably the cells (66) have substantially hexagonal geometric configurations when the stent is in a nominally expanded state.

16. The stent of claim 1, wherein each end of a connecting strut is an extension of an expansion strut that extends from contra-lateral sides of two opposing expansion strut pairs of the first and second expansion columns.

17. The stent of claim 1, wherein each expansion strut has at least one stair-step segment at one end of the expansion strut.

18. The stent of claim 1, wherein one expansion strut of an expansion strut pair has a stair-step segment at a proximal end and the other expansion strut of the expansion strut pair has a stair-step segment at a distal end.

19. The stent of claim 1 or 10, wherein the first and second expansion columns each forms a corrugated expansion ring,
and optionally, the third expansion column forms a corrugated expansion ring.

## Patentansprüche

1. Stent (10) im nicht aufgeweiteten Zustand, umfassend:
• eine erste aufweitbare Spalte (12), die einzelne aufweitbare Stege (14) umfasst, die eine Mehrzahl aufweitbarer Stegepaare (16) bilden, wobei sich zwei benachbarte aufweitbare Stegepaare (16) einen gemeinsamen Steg (18) teilen,
• eine zweite aufweitbare Spalte (20), die einzelne aufweitbahre Stege (22) umfasst, die eine Mehrzahl aufweitbarer Stegepaare (24) bilden, wobei sich zwei benachbarte aufweitbare Stegepaare (24) einen gemeinsamen Steg teilen,
• eine erste Verbindungsstegspalte (26), die eine Mehrzahl einzelner Verbindungsstege (28) umfasst, wobei jeder einzelne Verbindungssteg (28) ein Verlängerungsarm (30) eines einzelnen aufweitbaren Stegs (14) aus der ersten aufweitbaren Spalte (12) und ein Verlängerungsarm (32) eines einzelnen aufweitbaren Stegs (22) der zweiten aufweitbaren Spalte (20) ist,
**dadurch gekennzeichnet, dass** jeder Verbindungssteg (28) ein doppelt gestuftes Muster bildet.

2. Stent nach Anspruch 1, wobei mindestens ein Ende eines Verbindungsstegs (28) ein Verlängerungsarm eines aufweitbaren Stegs aus der ersten (12) oder zweiten (20) aufweitbaren Spalte ist.

3. Stent nach Anspruch 1, wobei jedes Ende eines Verbindungsstegs (28) ein Verlängerungsarm eines aufweitbaren Stegs ist, der sich von entgegengesetzten Seiten von zwei gegenüberliegenden aufweitbaren Stegepaaren (16, 24) der ersten (12) und zweiten (20) aufweitbaren Spalte aus erstreckt.

4. Stent nach Anspruch 1, wobei mindestens ein Ende eines Verbindungsstegs (28) ein Verlängerungsarm eines aufweitbaren Stegs ist, der sich von der entgegengesetzten Seite oder der gleichen Seite von einem von zwei gegenüberliegenden aufweitbaren Stegepaaren (16, 24) der ersten (12) und zweiten (20) aufweitbaren Spalte aus erstreckt.

5. Stent nach Anspruch 1, wobei jeder Verbindungssteg (28) eine Längsachse aufweist, die nicht senkrecht zu einer Längsachse des Stents (10) verläuft, oder
• im Wesentlichen senkrecht zu einer Längsachse des Stents (10) verläuft, oder
• deren Winkel im Wesentlichen schräg in Bezug auf eine Längsachse des Stents (10) verläuft, oder
• im Wesentlichen parallel zu einer Längsachse des Stents (10) verläuft.

6. Stent nach Anspruch 1, wobei jeder Verbindungssteg (28) eine Längsachse aufweist und im Wesentlichen jede Längsachse eines Verbindungsstegs (28) in der ersten Verbindungsspalte (26) parallel zu den anderen in der ersten Verbindungsspalte (26) verläuft.

7. Stent nach Anspruch 1, wobei jede Längsachse eines Verbindungsstegs (28) in einer Verbindungsstegspalte (26) parallel zu den Längsachsen weiterer Verbindungsstege in derselben Verbindungsstegspalte (26) in einem schrägen Winkel in Bezug auf eine Längsachse des Stents (10) verläuft.

8. Stufe nach Anspruch 1, wobei jeder Verbindungssteg (28) mindestens ein gestuftes Muster bildet, das mindestens einen im Wesentlichen waagerechten Abschnitt und mindestens einen im Wesentlichen schiefwinkligen Abschnitt umfasst.

9. Stent nach Anspruch 1, wobei jeder Verbindungssteg (28) ein gestuftes Muster mit mindestens einem im Wesentlichen waagerechten Abschnitt, mindestens einem im Wesentlichen schiefwinkligen Abschnitt und einem gekrümmten Abschnitt bildet, der den im Wesentlichen waagerechten Abschnitt und den im Wesentlichen schiefwinkligen Abschnitt verbindet, wobei der gekrümmte Abschnitt vorzugsweise mindestens ein oder zwei Krümmungsradien aufweist,
ein Krümmungsradius an einer Verbindungsstelle zwischen einem im Wesentlichen waagerechten Abschnitt und einem im Wesentlichen schiefwinkligen Abschnitt einen Krümmungsradius mit einem großen Winkel oder einen Krümmungsradius mit einem kleinen Winkel aufweist.

10. Stent nach Anspruch 1, ferner umfassend:
• eine dritte aufweitbare Spalte (34), die einzelne aufweitbare Stege (36) umfasst, die eine Mehrzahl aufweitbarer Stegepaare (38) bilden, wobei sich zwei benachbarte aufweitbare Stegepaare einen gemeinsamen Steg teilen,
• eine zweite Verbindungsstegspalte (40), die eine Mehrzahl einzelner Verbindungsstege (42) umfasst, wobei jeder einzelne Verbindungssteg (42) ein Verlängerungsarm (43) eines einzelnen aufweitbaren Stegs (22) aus der zweiten aufweitbaren Spalte (20) und ein Verlängerungsarm (43) eines einzelnen aufweitbaren Stegs (36) der dritten aufweitbaren Spalte (34) ist, und gegebenenfalls
• eine Mehrzahl von aufweitbaren Spalten, die durch eine Mehrzahl von Verbindungsstegspalten verknüpft sind, wobei jeder Verbindungssteg in einer Verbindungsstegspalte eine Längsachse aufweist, wobei die Längsachse der Verbindungsstege in einer Verbindungsstegspalte in einer entgegengesetzten Richtung zur Längsachse der Verbindungsstege in einer benachbarten Verbindungsstegspalte verläuft.

11. Stent nach Anspruch 10, wobei jeder Verbindungssteg (28) in der ersten Verbindungsstegspalte (26) eine erste Längsachse aufweist und jeder Verbindungssteg (42) in der zweiten Verbindungsstegspalte (40) eine zweite Längsachse aufweist, die nicht parallel zur ersten Längsachse verläuft.

12. Stent nach Anspruch 10, wobei die Längsachse von jedem Verbindungssteg in einer Verbindungsstegspalte einen schrägen Winkel in Bezug auf die Längsachse des Stents aufweist, wobei jede Längsachse der Verbindungsstege in einer Verbindungsstegspalte einen schrägen Winkel in einer entgegengesetzten Richtung zu einem schrägen Winkel jeder Längsachse des Verbindungsstegs in einer benachbarten Verbindungsstegspalte aufweist.

13. Stent nach Anspruch 10, wobei ein schräger Winkel der Längsachse jedes Verbindungsstegs in einer Verbindungsstegspalte, die in einem abwechselnden Muster eine entgegengesetzte Richtung zu einem schrägen Winkel der Längsachse jedes Verbindungsstegs in einer benachbarten Verbindungsstegspalte von einer Mehrzahl von aufeinander folgenden Verbindungsstegspalten aufweist.

14. Stent nach Anspruch 1, wobei die erste aufweitbare Spalte (12), die zweite aufweitbare Spalte (20) und die erste Verbindungsstegspalte- (26) eine Mehrzahl Zellen (66) definieren.

15. Stent nach Anspruch 14, wobei die Zellen eine asymmetrische Geometrie aufweisen, oder
die Zellen (66) über den gesamten Stent geometrische Formen mit gleichem Abstand aufweisen, die Zellen (66) vorzugsweise im Wesentlichen sechseckige geometrische Anordnungen aufweisen, wenn sich der Stent in einem planmäßig aufgeweiteten Zustand befindet.

16. Stent nach Anspruch 1, wobei jedes Ende eines Verbindungsstegs eine Verlängerung eines aufweitbaren Stegs ist, die sich von entgegengesetzten Seiten von zwei gegenüberliegenden aufweitbaren Stegepaaren der ersten und zweiten aufweitbaren Spalte aus erstreckt.

17. Stent nach Anspruch 1, wobei jeder aufweitbare Steg mindestens einen gestuften Abschnitt an einem Ende des aufweitbaren Stegs aufweist.

18. Stent nach Anspruch 1, wobei ein aufweitbarer Steg eines aufweitbaren Stegepaars einen gestuften Abschnitt an einem proximalen Ende aufweist und der andere aufweitbare Steg des aufweitbaren Stegepaars einen gestuften Abschnitt an einem distalen Ende aufweist.

19. Stent nach Anspruch 1 oder 10, wobei die erste und zweite aufweitbare Spalte jeweils einen gewellten aufweitbaren Ring bilden,
und gegebenenfalls die dritte aufweitbare Spalte einen gewellten aufweitbaren Ring bildet.

## Revendications

1. Stent (10) dans un état non déployé comprenant :
• une première colonne d'extension (12) comprenant des tiges d'extension individuelles (14) formant une pluralité de paires de tiges d'extension (16), dans lesquelles deux paires de tiges d'extension adjacentes (16) partagent une tige commune (18) ;
• une deuxième colonne d'extension (20) comprenant des tiges d'extension individuelles (22) formant une pluralité de paires de tiges d'extension (24) dans lesquelles deux paires de tiges d'extension adjacentes (24) partagent une tige commune ;
• une première colonne de tiges de liaison (26) comprenant une pluralité de tiges de liaison individuelles (28), dans lesquelles chaque tige de liaison individuelle (28) est un bras d'extension (30) d'une tige d'extension individuelle (14) depuis la première colonne d'extension (12), et un bras d'extension (32) d'une tige d'extension individuelle (22) de la deuxième colonne d'extension (20),
**caractérisé en ce que** chaque tige de liaison (28) forme un escalier à deux marches.

2. Stent selon la revendication 1, dans lequel au moins une extrémité d'une tige de liaison (28) est un bras d'extension d'une tige d'extension depuis la première (12) ou la deuxième (20) colonne d'extension.

3. Stent selon la revendication 1, dans lequel chaque extrémité d'une tige de liaison (28) est un bras d'extension d'une tige d'extension, se déployant depuis les faces contre-latérales de deux paires (16, 24) de tiges d'extension opposées des première (12) et deuxième (20) colonnes d'extension.

4. Stent selon la revendication 1, dans lequel au moins une extrémité d'une tige de liaison (28) est un bras d'extension d'une tige d'extension se déployant depuis la face contre-latérale ou la face ipsilatérale d'une de deux paires (16, 24) de tiges d'extension opposées des première (12) et deuxième (20) colonnes d'extension.

5. Stent selon la revendication 1, dans lequel chaque tige de liaison (28) présente un axe longitudinal qui est non-perpendiculaire à un axe longitudinal du stent (10), ou
est sensiblement perpendiculaire à un axe longitudinal du stent (10), ou
présente un angle sensiblement diagonal par rapport à un axe longitudinal du stent (10), ou
est sensiblement parallèle à un axe longitudinal du stent (10).

6. Stent selon la revendication 1, dans lequel chaque tige de liaison (28) présente un axe longitudinal, et essentiellement tout axe longitudinal d'une tige de liaison (28) de la première colonne de liaison (26) est parallèle aux autres dans la première colonne de liaison (26).

7. Stent selon la revendication 1, dans lequel chaque axe longitudinal d'une tige de liaison (28) d'une colonne de tiges de liaison (26) est parallèle aux axes longitudinaux des autres tiges de liaison de la même colonne de tiges de liaison (26) à un angle diagonal par rapport à un axe longitudinal du stent (10).

8. Étape selon la revendication 1, dans laquelle chaque tige de liaison (28) forme au moins un escalier comprenant au moins un segment sensiblement horizontal et au moins un segment sensiblement à angle oblique.

9. Stent selon la revendication 1, dans lequel chaque tige de liaison (28) forme un escalier avec au moins un segment sensiblement horizontal, au moins un segment sensiblement à angle oblique, et une section incurvée qui relie le segment sensiblement horizontal et le segment sensiblement à angle oblique, la section incurvée ayant au moins un ou deux rayons de courbure, de préférence
un rayon de courbure à une jonction entre un segment sensiblement horizontal et un segment sensiblement à angle oblique présente un large rayon angulaire de courbure ou un rayon angulaire étroit de courbure.

10. Stent selon la revendication 1, comprenant en outre
• une troisième colonne d'extension (34) comprenant des tiges d'extension individuelles (36) formant une pluralité de paires de tiges d'extension (38), dans lesquelles deux paires de tiges d'extension adjacentes partagent une tige commune ;
• une deuxième colonne de tiges de liaison (40) comprenant une pluralité de tiges de liaison individuelles (42), dans lesquelles chaque tige de liaison individuelle (42) est un bras d'extension (43) d'une tige d'extension individuelle (22) depuis la deuxième colonne d'extension (20), et un bras d'extension (43) d'une tige d'extension individuelle (36) de la troisième colonne d'extension (34) et en option,
• une pluralité de colonnes d'extension couplées par une pluralité de colonnes de tiges de liaison, chaque tige de liaison d'une colonne de tiges de liaison ayant un axe longitudinal, l'axe longitudinal des tiges de liaison d'une colonne de tige de liaison s'étendant dans une direction opposée par rapport à l'axe longitudinal de tiges de liaison dans une colonne de tiges de liaison adjacente.

11. Stent selon la revendication 10, dans lequel chaque tige de liaison (28) de la première colonne de tiges de liaison (26) présente un premier axe longitudinal et chaque tige de liaison (42) de la deuxième colonne de tiges de liaison (40) présente un second axe longitudinal qui est non-parallèle au premier axe longitudinal.

12. Stent selon la revendication 10, dans lequel l'axe longitudinal de chaque tige de liaison d'une colonne de tiges de liaison présente un angle diagonal par rapport à l'axe longitudinal du stent, chaque axe longitudinal des tiges de liaison d'une colonne de tiges de liaison ayant un angle diagonal dans une direction opposée par rapport à un angle diagonal de chaque axe longitudinal de la tige de liaison d'une colonne de tiges de liaison adjacente.

13. Stent selon la revendication 10, dans lequel un angle diagonal de l'axe longitudinal de chaque tige de liaison d'une colonne de tiges de liaison présentant une direction opposée alternativement par rapport à un angle diagonal de l'axe longitudinal de chaque tige de liaison d'une colonne de tiges de liaison adjacente d'une pluralité de colonnes de tiges de liaison consécutives.

14. Stent selon la revendication 1, dans lequel la première colonne d'extension (12), la seconde colonne d'extension (20) et la première colonne de tiges de liaison (26) définissent une pluralité de cellules (66).

15. Stent selon la revendication 14, dans lequel les cellules présentent une géométrie asymétrique ; ou
les cellules (66) présentent des formes géométriques espacées uniformément à travers le stent, de préférence les cellules (66) présentent des configurations géométriques sensiblement hexagonales lorsque le stent se trouve dans un état essentiellement déployé.

16. Stent selon la revendication 1, dans lequel chaque extrémité d'une tige de liaison est une extension d'une tige d'extension qui s'étend depuis les faces contre-latérales de deux paires de tiges d'extension opposées des première et deuxième colonnes d'extension.

17. Stent selon la revendication 1, dans lequel chaque tige d'extension présente au moins un segment en marche d'escalier à une extrémité de la tige d'extension.

18. Stent selon la revendication 1, dans lequel une tige d'extension d'une paire de tiges d'extension présente un segment en marche d'escalier à une extrémité proximale et l'autre tige d'extension de la paire de tiges d'extension comprend un segment en marche d'escalier à une extrémité distale.

19. Stent selon la revendication 1 ou 10, dans lequel les première et deuxième colonnes d'extension forment chacune un anneau d'extension cannelé,
et en option, la troisième colonne d'extension forme un anneau d'extension cannelé.
